# EUROPEAN PATENT APPLICATION

(11) **EP 2 732 796 A1**
(43) Date of publication of application: **21.05.2014**
(21) Application number: 12193489.7
(22) Date of filing: 20.11.2012
(51) Int. Cl.: A61F 2/24

(54) **Mitral valve replacement system**

(71) Applicant: Nakostech Sarl, 75005 Paris (FR)
(72) Inventor: Pouletty, Philippe, 75005 Paris (FR)
(74) Representative: Hepp Wenger Ryffel AG

(57) **Abstract**

The invention concerns a minimally invasive valve repair system (2), in particular a mitral valve repair system (2) or a tricuspid valve repair system. The system comprises a valve component (3), in particular a mitral valve component (3) or a tricuspid valve component, holding or adapted to hold a valve (4), in particular a mitral valve (4) or a tricuspid valve, respectively. The system (2) comprises a linker-component (6) by means of witch the valve component (3) is linked or linkable to an anchor support (5), in particular an in particular an aortic valve or aortic anchor support (5) or a pulmonic valve or pulmonary artery anchor support, respectively.

## Description

The present invention relates to a valve repair system according to the preamble of claim 1. The invention further relates to a delivery device for repair systems, in particular to a delivery device according to the preamble of claim 9 and a method for delivering the system according to the preamble of claim 12.

The mitral valve also known as bicuspid valve or left atrioventricular valve lies between the left atrium and the left ventricle in the heart. It is a dual-flap valve that opens during left ventricular diastole and prevents backflow from the ventricle into the left atrium during left ventricular systole. Several disorders are associated with a malfunctioning mitral valve, Stenosis occurs when the valve is too tight for blood flow in the left ventricle as a result of calcium nodes, leaflet thickening and/or reduced leaflet mobility. Mitral regurgitation occurs when the mitral valve is too loose and the blood can leak back into the left atrium during systole. The diseases can have different causes such as a congenitally abnormal mitral valve, infections or a bacterial endocarditis. More often are age-related malfunctions caused by degenerative valves.

The tricuspid valve also known as the right atrioventricular valve lies between the right atrium and the right ventricle. The tricuspid valves usually has three leaflets and three papillary muscles however, the number of leaflets may be different from three and can change during one's lifetime. The tricuspid valve opens during right ventricular diastole and prevents backflow from the ventricle into the right atrium during right ventricular systole.

Several disorders are associated with a malfunctioning tricuspid valve, regurgation, the backflow of blood from the right ventricle in the right atrium, occurs if the tricuspid valve functions imperfect. Stenosis occurs when the valve is too tight. The diseases can be caused by infections, e.g. *S. aureus* infection. Other causes are affection by rheumatic fever or congenital apical displacement.

A conventional mitral or tricuspid valve treatment is an open heart surgery for surgical replacement with valve prosthesis. Such operations are invasive and can expose patients to risks such as bleeding or infection. It is therefore a need to provide an embodiment for a minimal invasive valve repair.

US 2012/0010700 A1 describes expandable prosthetic valves introducible into the patient's body via transvascular or transapical approaches. Before the prosthetic valve is installed, a hole is pierced in one of the native leaflets of the mitral valve. The piercing is performed mechanically or with a laser tool. Afterwards, the expandable seating of a prosthetic valve is positioned in the hole. The expandable seating is expanded, thereby opening the hole and wedgingly locking the seating within. Optionally the expansion can be effected using an expansion arrangement comprising a balloon. As the valve expands the hole widens and forces the outer edge of the first, pierced leaflet against the second leaflet, thereby closing the native orifice.

A procedure in which a hole is formed in the native leaflet can cause problems as the leaflet might separate during hole widening. This can result in a badly locked or even unlocked prostethic valve. It might also occur that the native orifice doesn't close well after expanding of the expandable seating. As a result, a backflow of blood during systole isn't avoided.

It is an object of the invention to overcome the disadvantages of the known, in particular to provide an expandable mitral valve replacement system with a reliable locking system.

This is accomplished with the features of characterising parts of claims 1, 9, and 12 according to the invention. A valve repair system, comprising a valve component, in particular a mitral valve component or a tricuspid valve component, is holding or adapted to hold a valve, in particular a mitral valve or a tricuspid valve, respectively. The left heart system comprises a linker-component for linking the mitral valve component to an aortic anchor support or to an aortic valve anchor support. The right heart system comprises a linker-component for linking the tricuspid valve component to a pulmonary artery anchor support or a pulmonic valve anchor support.

The anatomy around the mitral valve or the tricuspid valve makes positioning and long term maintenance of an artificial valve component difficult. There are no ducts providing possibilities for component with e.g. a radial force fixation. Therefore, a fixation to an anchor support in the aorta/aortic annulus or the pulmonary artery/pulmonary artery annulus, respectively is favourable. The aorta or the aortic valve annulus, as known by a person skilled in the art, provides dependable anchoring possibilities for stents or stent-like structures. The pulmonary artery or the pulmonary artery annulus provides similar conditions. By fixating the valve component to an anchor support located around the aortic or pulmonic valve, a reliable fixation of the mitral or tricuspid valve respectively is provided. The anchor support provides maintaining the valve component in the correct position and prevents paravalvular leaks or slippage of the valve into the atrium or into the ventricule.

The linker-component is preferably constructed as an arm extending from the anchor support towards the valve component within the ventricule, or extending from the ascending aorta/pulmonary artery and the left/right atrium. In another form the linker component extends through the aorta wall/pulmonary artery wall from the anchor support towards the valve component.

The arm is linked at one end to the anchor support and at the other end to the valve component. The main body of the linker-component is in the ventricle when attached to the linkable parts. The linked valve component is fixated and thereby prevented from rotational and axial movement. With an external anchoring at an aortic anchoring system/pulmory artery anchoring system, the valve component features the same fixation reliability as an aortic valve stent/pulmonary valve stent. It is also possible to construct the linker-component as a two-arm or multiple-arm embodiment. As one arm is shown to provide a reliable fixation, it is the preferred form for obvious reasons such as costs and simplicity.

In a preferred embodiment, the valve component comprises a stent component. The stent component can either be a mitral valve stent or a tricuspid valve stent. Stents are very reliable in long term housing of artificial valves.

Alternatively the valve component comprises a simple element like e.g. an annular fixation ring holding the artificial valve or a balloon system holding the valve leaflets.

The anchor support can either be a pre-introduced valve stent from an antecedent aortic valve/pulmonary valve replacement or it can be introduced simultaneously with, or shortly before or after, the valve component is introduced. The introduced anchor support can either house a valve or not. The anchor support without a valve may be designed as a valveless anchor support. The anchor support without a valve may also be designed as an annulus fixation element adapted to sit onto the annulus. The fixation element can either sit in blood flow direction above the anchor support if the anchor linker is within the ventricule or below if the linker goes from the aorta/pulmonary artery to the atrium.

The linker-component can either be linked between the anchoring support and the valve component outside the body or after introducing of the valve components into the patient.

In a preferred embodiment of the invention, the valve repair system as described above comprises an anchor support. The anchor support is part of the system and is either introduced in the same step or sequentially with the artificial valve. In an especially preferred embodiment, the anchoring support is placed first and the valve is placed second.

Another possibility is to place the valve first and the valve anchor support second. The linker-component is introduced either with the anchor support or with the valve component. The anchor support and the valve component might be pre-linked with the linker component outside the body.

Otherwise, the different parts are linked with the linker component after delivering. A further alternative is that the linker-component is introduced separately and linked to the pre-introduced valve component and anchoring support.

In an especially preferred embodiment of the invention, the anchor support is an valve stent. This embodiment is desirable in case of a dual aortic and mitral or pulmonary and tricuspid valve disease. With the provided embodiment, only one same-day surgical procedure or one same hospitalization for minimal invasion is needed to fix both valves. This results in lower costs and saves time.

Also in patients who already have an aortic/pulmonary artery valve stent due to a previous medical intervention, the valve stent can be used as an anchor support. The arms of the anchoring structures of the aortic/pulmonary artery valve stent can serve as fixation structures for the linking-component. In case the aortic/pulmonary artery stent comprises anchoring structures without arms other fixation points are conceivable such as e.g. a crown or a hook, if any.

In another preferred embodiment of the invention, the anchor support is a valveless stent or an annulus fixation element adapted to sit onto the annulus. The fixation element can either sit in blood flow direction above the anchor support if the anchor linker is within the ventricule or below if the linker goes from the aorta/pulmonary artery to the atrium.

This embodiment is desirable in case of a healthy aortic/pulmonary artery valve. Especially in eldery patients who have only a mitral/tricuspid valve disease and a contraindication to surgery, this minimal invasive approach is desired. But also in younger patients the valveless stent as an anchoring support provided by minimal invasive treatment might be the preferred option. The anchoring systems provide a reliable fixation of the mitral/tricuspid valve component.

One form of a valveless aortic/pulmonary artery stent is a supra annulus stent with a thin but rigid linear or non-linear arm descending between native valve leaflets into the ventricule. Another form would be an embodiment with two or multiple arms extending in between native valve leaflets to the ascending aorta/pulmonary artery. In the latter form one of the arms has at its ventricular end an attachment site for an anchoring arm extending towards the mitral/tricuspid valve stent. In another additional form an arm extends through the wall between the ascending aorta/pulmonary artery and the atrium.

In a preferred embodiment of the invention, the anchor support is adopted to be positioned onto the annulus and/or the aorta/pulmonary artery. The preferred possibilities for stent anchoring, as known to a person skilled in the art, are embodiments which provide radial force onto the valve annulus or provide one or several hooks onto the annulus, or a stent with an arch to put radial force onto the ascending aorta/pulmonary artery. Such structures have shown to provide a reliable fixation for aortic/pulmonary artery stents.

It is also possible to fixate the anchor valve in another way, e.g. with fixation structures penetrating the duct wall. Arms providing radial force onto the annulus and/or aorta/pulmonary artery are associated with minimal intricacies together with maintaining the desired position reliable over time. Therefore, arms are the preferred fixation structure for the described anchoring system of the invention.

The valve component and/or the anchor support are preferably made of nitinol. Nitinol is known for its shape memory and its high biocompatibility. Therefore, nitinol is often used for stents or other body implants. Other materials for the mitral stent and/or aortic valve anchor support are biocompatible metals or metal alloys. It is also possible to use biocompatible polymer stents.

In a preferred embodiment, the valve is made of biological material such as autologous or animal pericardium or leaflets. Possible non autologous pericardium or leaflets are horse, bovines, veal, porcine or other compatible animal pericardium or leaflets. Another possibility is to use synthetic valves in combination with the replacement system. However, biological valves have shown to reduce complications e.g. with thromboembolism and are therefore favourable.

The invention further relates to a delivery device for minimal invasive delivering a valve repair system. The delivery device comprises a repair system comprising a valve component holding or adopted to hold a valve and an aortic/pulmonary artery anchor support and a component that links or is linkable the valve component and the aortic anchor support. The device further comprises an outer sheath and a guide wire tubing and at least one stent holder constructed to house at least parts of the valve repair system.

The delivery device is designed for minimal invasive introduction into a body. The introduction of the valve repair system is possible at an inserting point on the patient's body. The device can be introduced either by vascular route and/or intracardiac route. The vascular route includes trans-venous, trans-septal, transfemoral, subclavian, and transaortic. The intracardiac route includes transapical, transatrial, and transseptal. After inserting, the replacement valve system is releasable at a designated body part.

The device is guided with a guide wire. The guide wire is preferably housed inside the guide wire tubing. When the delivery device reaches the designated body part, the outer sheath is moved relatively to the stent holder and the repair system is able to be deployed. A preferred delivery device is a catheter. Catheters are successfully used for the introduction of valve components as known in the art.

In a preferred embodiment, the delivery device is one delivery catheter constructed to introduce the whole system simultaneously. The catheter offers space for all parts of the replacement system. In a first step the anchor support is able to be deployed in the aorta/pulmonary artery and in a second step the valve component is able to be deployed in the left/right atrium.

The aortic anchor support and the valve component are arranged in the catheter for separate deployment. The catheter may comprise two separate stent holders constructed to accept either the valve component or the anchoring support. In another preferred embodiment, the catheter comprises only one stent holder constructed to accept the whole repair system. The anchor support as well as the valve component have to be crimped prior to the acceptation by the at least one stent holder. When the anchoring support and the valve component are accepted by the stent holder, the linker component is either linked to the anchoring support or to the valve component. The linker component is linked to the not yet linked part of the repair system after deployment at the designated body part. In another preferred embodiment, the linker component links the anchoring support and the valve component before crimping. The different previously described routes are options for a one catheter delivery device.

In another preferred embodiment, the delivery device consists of several delivery catheters constructed to introduce the system sequentially. With a first catheter the anchor support is guided and able to be deployed in the aorta/pulmonary artery. With a second catheter the valve component is guided and able to be deployed in the left/right atrium. Each of the catheters comprise a stent holder, either to accept the anchoring support or to accept the valve comonent. Crimping of the support and the valve component has to be performed before being accepted by the stent holder. The linker component is either linked to the anchoring support or to the valve component before crimping. After deployment at the designated body part, the linker component is linked to the not yet linked part of the repair system. The different previously described routes are all options for a several catheter delivery device too.

The invention further relates to a method for minimal invasive introducing a mitral/tricuspid valve repair system. The system comprises a valve component, holding or adopted to hold a valve, and an aortic/pulmonary artery anchor support and a component that links or is linkable the valve component and the anchor support with a delivery device. The method involves the steps:
a) Introcucing a delivery device at an inserting point and guiding the delivery device to a designated body part
b) releasing and fixing the anchor support at an aortic/pulmonary artery anchoring site
c) releasing and deployment of the valve component valve
d) linking the anchor support and the valve component,
Wherein step d) is performed before step a), between step a) and b), between step b) and c), or after step c), and wherein step b) is performed before or after step c).

It is possible to link the linker component either to the anchor support or to the valve component outside the body. Linking to the other part of the repair system is performed after deployment of the whole system inside the body. In another embodiment the anchoring system and the valve component are linked with linker component outside the body.

Further advantages and details of the invention are illustrated in the drawings and are hereinafter described. Schematically shown are:
- Figure 1: Left side of a human heart with the mitral valve replacement system in position
- Figure 2a-g: Different steps in the delivery process
- Figure 3: Alternative aortic anchor support
- Figure 4: Alternative aortic anchor support
- Figure 5: Alternate linker-component
- Figure 6: Linking system

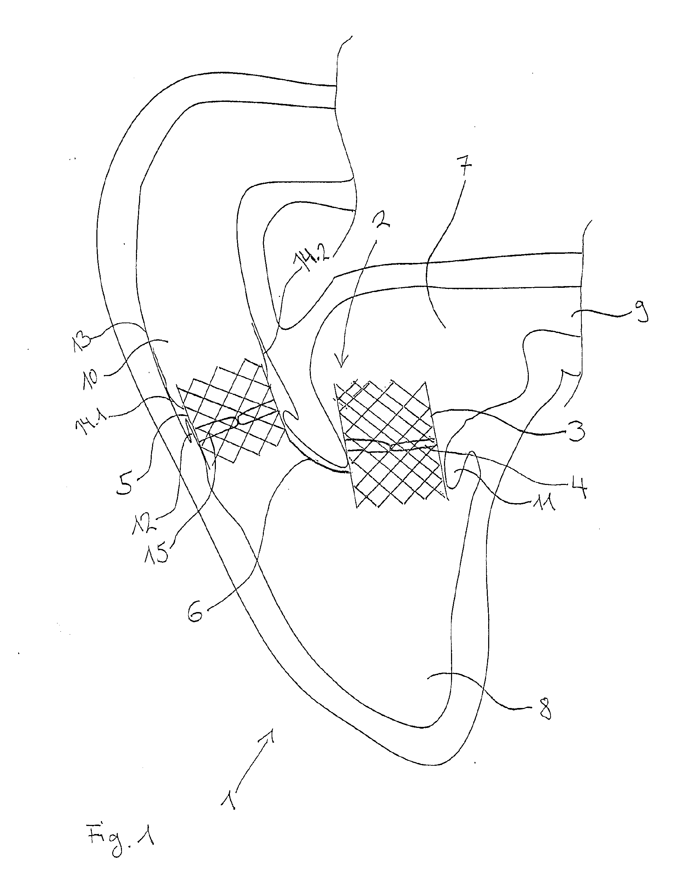

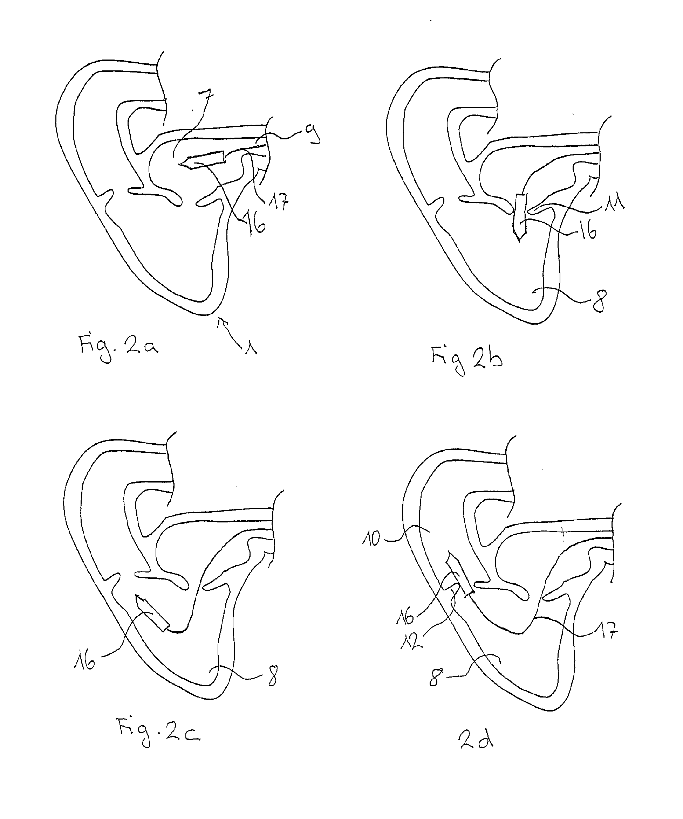

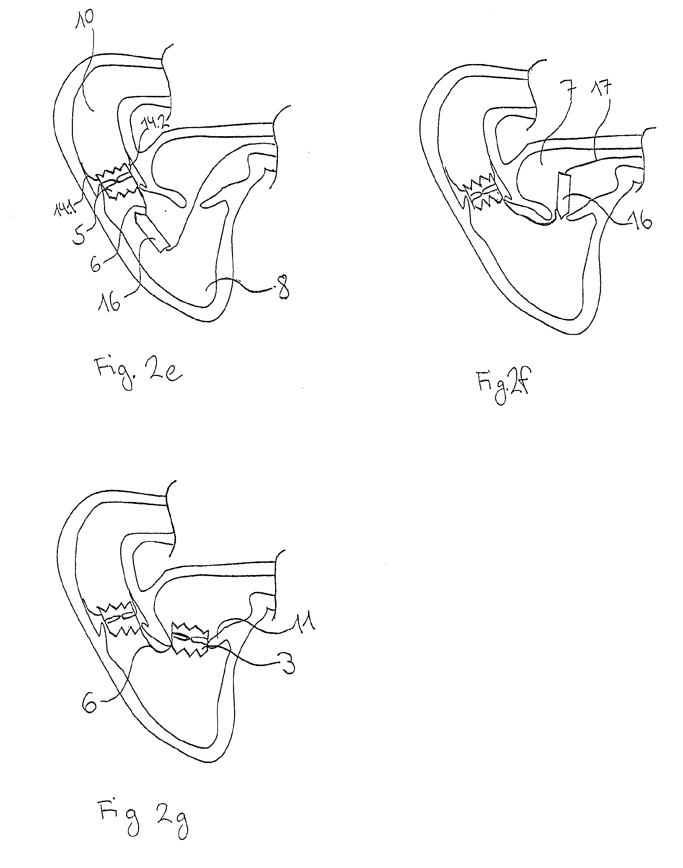

Figure 1 shows a longitudinal section of the left side of a human heart 1 with a dual aortic and mitral valve repair system 2 in position. The system comprises a mitral valve stent 3 housing a mitral valve 4, an aortic anchor support 5 and a schematic linker component 6. A coil can be part of the linker or a V shape linker can be used. The linker ensures stability of the mitral valve, while allowing some flexibility during diastole and systole. The aortic anchor support 5 is constructed as an aortic stent housing an aortic valve component 15. In an alternative embodiment, valve component 15 may be omitted if there is no need for aortic valve replacement. The aortic anchor support 5 may then be placed higher in the ascending aorta 10. The aortic anchoring stent 5, the linker component 6, and the mitral valve stent 4 are all made of nitinol. Both stents 4,5 have a cylindrical shape out of nitinol strings forming a lattice structure. The replacement system is delivered with an intracardiac delivery device (see fig. 2) simultaneously. The delivery device constructed as a catheter is introduced into the left atrium 7 through the pulmonary vein 9. The delivery device is then guided through the orifice formed by the native mitral valve leaflets 11 into the left ventricle 8. From the left ventricle 8 the delivery device is further guided through the native aortic valve leaflets 12 into the ascending aorta 10. In the ascending aorta 10, the aortic valve stent 5 with the aortic valve stent anchoring arms 14.1,14.2, and the linker-component 6 are deployed. The linker component is linked to the ventricular end of the anchoring arm 14.2 which lies at the aortic wall 13 next to the left atrium 7. The other end of the linker component 6 is not yet linked to the mitral valve stent at this point. The anchoring arms 14.1,14.2 provide radial force onto the aortic wall 13 which provides fixation of the aortic valve stent 5. The delivery device is then guided back to the the area of the native mitral valve leaflets 11. There, the mitral valve stent 3 housing the mitral valve component 4 is deployed and brought into the right position to replace the native mitral valve leaflets 11. In this position, the mitral valve stent 3 is attached to the free end of the linker-component 6. Hooks or female / male components can be used to easily fix the linker between the mitral and aortic components. The dual aortic and mitral valve replacement system 2 is now in its final position and the delivery device is pulled out of the body.

Figure 2a shows the situation when a delivery catheter 16 is introduced into the left side of the heart 1 through the pulmonary vein 9. The delivery catheter is guided along a guide wire 17 in the left atrium 7. Afterwards, the delivery catheter 16 is guided forward until reaching the point shown in Fig 2b. The catheter 16 enters the left ventricle 8 through the orifice formed by the native mitral valve leaflets 11. Fig. 2c shows the situation when the catheter 16 is completely in the left ventricle 8. From the left ventricle 8 the delivery catheter 16 is guided with the guide wire 17 through the orifice formed by the native aortic leaflets 12 into the aorta 10, shown in Fig. 2d. This is the position in which the aortic anchoring support 5 is released from the delivery device 16. For releasing the anchoring support 5, the outer sheath of the catheter 16 is moved axially relative to the stent holder, housing the anchoring support 5. The anchoring support 5 expands automatically after releasing from the catheter 16. Fig. 2e shows the situation when the aortic anchoring support 5 is deployed and anchored with the anchoring arms 14.1,14.2 in the aorta 10. The anchoring arms 14.1,14.2 deploy automatically from the cylindrical anchoring support 5 and fixate the anchoring support 5 through putting radial force on the ascending aorta 10. The catheter 16 is at this point again completely in the left ventricle 8. The linker component 6 is pre-linked outside the body to the ventricular end of the anchoring arm 14.2. The other end of the linker component 6 is still housed within the delivery device 16. The catheter 16 is further pulled back with the guide wire 17 until the position showed in fig. 2f which shows the catheter 16 again at least partly in the left atrium 7. Here, the mitral valve stent 3 is released. The releasing of the mitral valve stent 3 is similar to that of the anchoring support 5. The outer sheath is moved axially relative to the stent holder housing the mitral valve stent 3. Like the anchoring support 5 the mitral valve stent 3 expands automatically. After the mitral valve stent 3 is fully expanded, the linker-component 6 is attached to the mitral valve stent 3 Fig. 2g shows the situation when the mitral valve stent 3 is deployed and installed at the position of the native mitral valve leaflets 11. The mitral valve stent 3 is linked to the linker component 6 and therefore fixed and prevented from slippage.

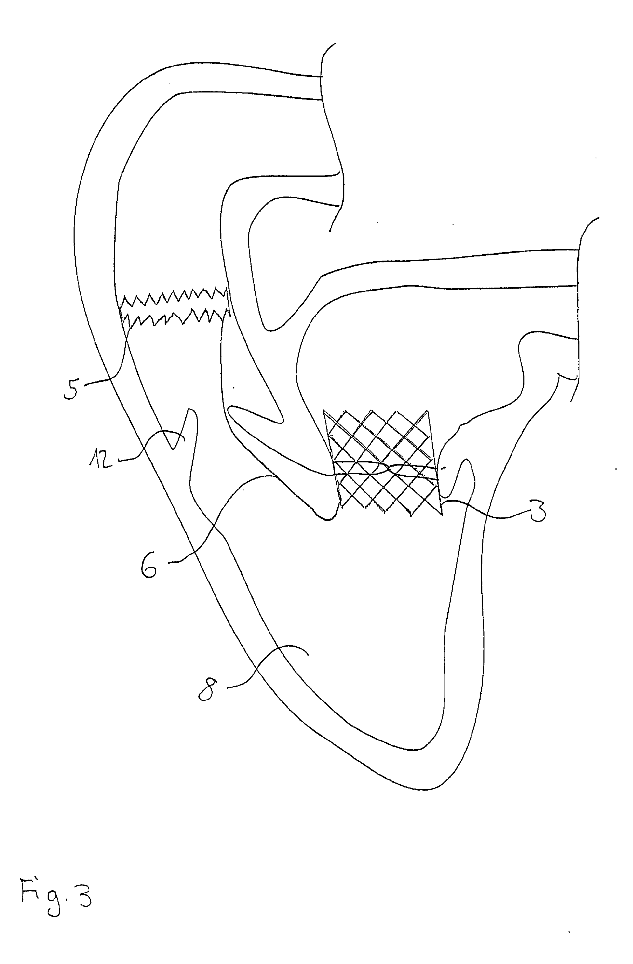

Figure 3 shows an alternative replacement system with an anchoring support 5 constructed as a valveless supra annulus stent. The linker component 6 is descending between the native aortic leaflets 12 into the left ventricle 8 and towards the mitral valve stent 3.

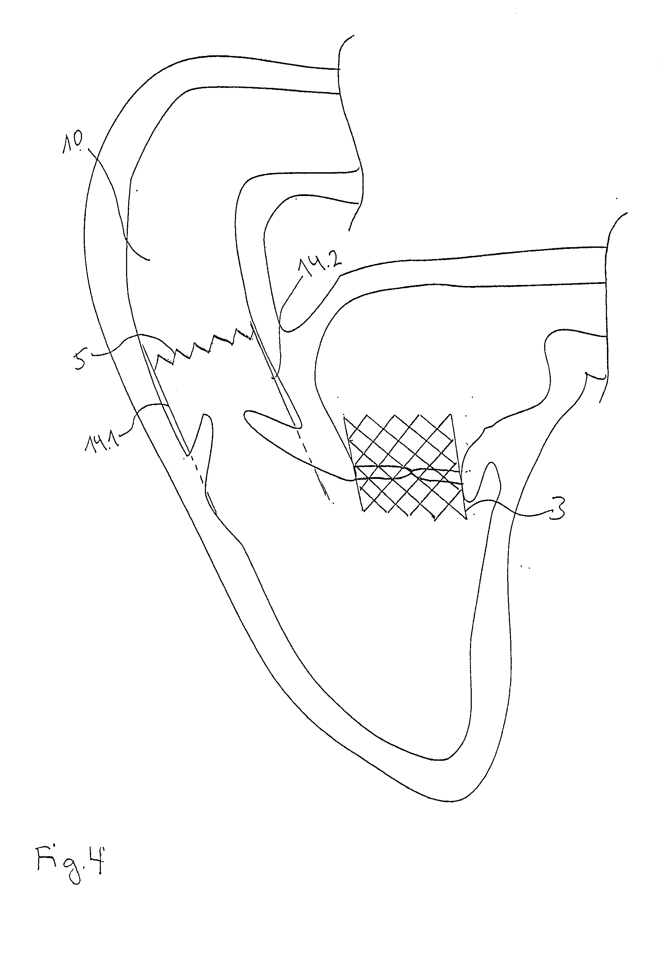

Figure 4 shows a further alternative replacement system with an anchoring support 5 constructed as two linear anchoring arms 14.1 and 14.2 which extend in between native valve leaflets 12 to the ascending aorta 10. One anchoring arm 14.2 has at the ventricular end a linking component 6 extending towards the mitral valve stent 3.

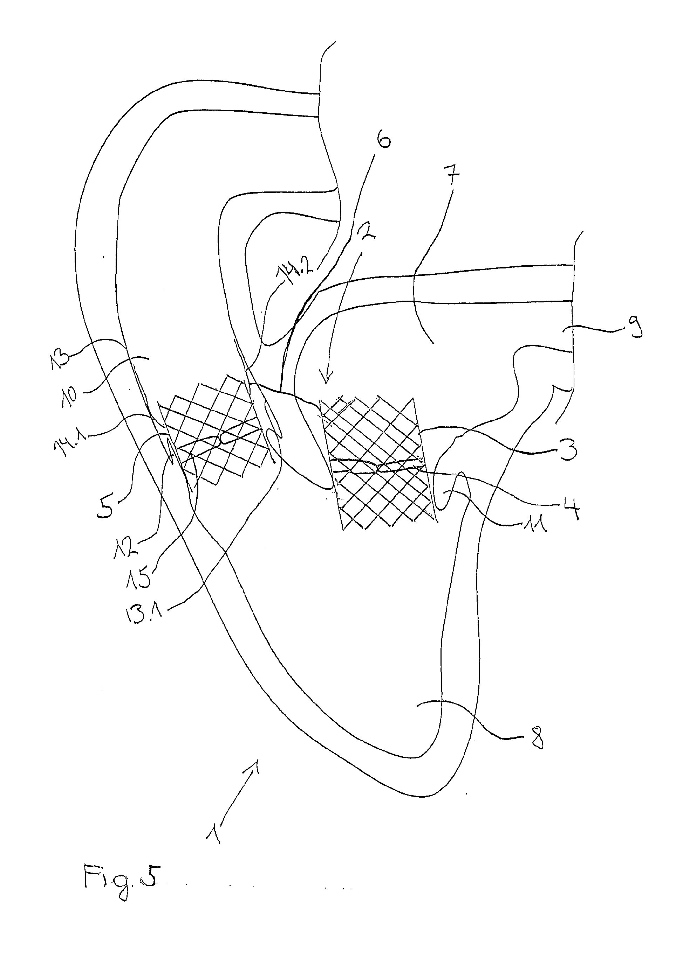

Figure 5 shows an alternative linker component 6. The linker component extends through the aortic wall 13.1 separating the aorta and the left atrium.

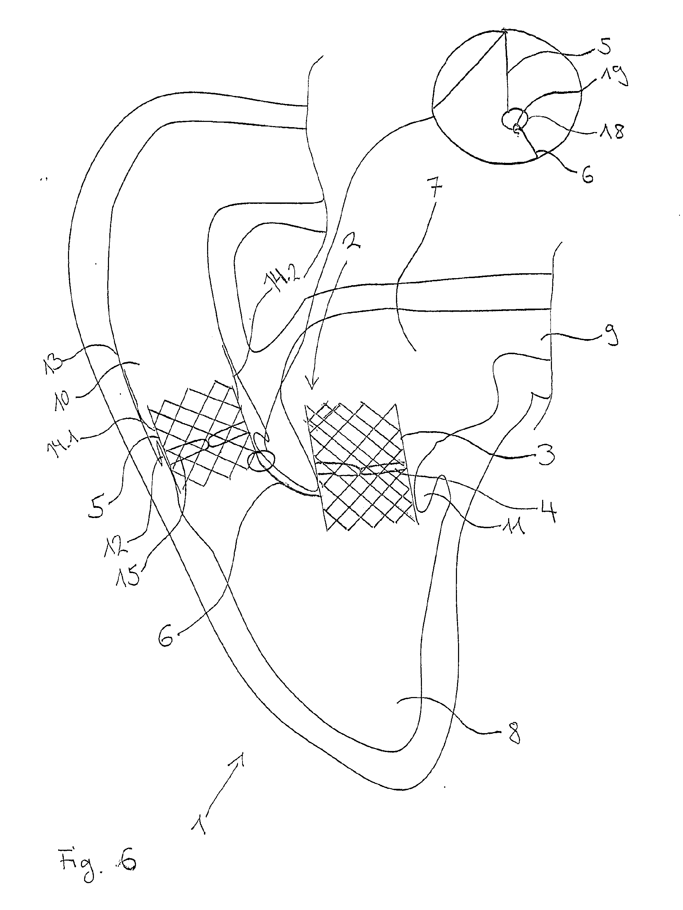

Figure 6 shows a detailed view of the fixation of the linker component 6 to the aortic anchor support 5. The aortic anchor support comprises a female locking element 18. The linker component 6 comprises a male locking element 19 adopted to provide a reliable locking in combination with the female locking element 18.

## Claims

1. Minimally invasive valve repair system (2), in particular a mitral valve repair system (2) or a tricuspid valve repair system , comprising a valve component (3), in particular a mitral valve component (3) or a tricuspid valve component, holding or adapted to hold a valve (4), in particular a mitral valve (4) or a tricuspid valve
**characterized in that**
the system comprises a linker-component (6) by means of which the valve component (3) is linked or linkable to an anchor support (5), in particular an aortic valve or aortic anchor support (5) or a pulmonic valve or pulmonary artery anchor support.

2. The valve repair system (2) of claim 1, wherein the valve component comprises a stent (3), in particular a mitral valve stent (3) or a tricuspid valve stent.

3. The valve repair system (2) of claim 1 or 2, wherein the system further comprises an anchor support (5), in particular an aortic valve anchor support or aortic anchor support (5) or a pulmonic valve or pulmonary artery anchor support.

4. The valve repair system (2) of claim 3, wherein the anchor support (5) is a valve stent, in particular an aortic valve stent or a pulmonic valve stent.

5. The valve repair system (2) of claim 3 or 4, wherein the anchor support (5) is a valveless stent or an annulus fixation element adapted to sit onto the annulus of the aorta or of the pulmonary artery.

6. The valve repair system (2) of any of the claims 3-5, wherein the anchor support (5) is adapted to be positioned onto the aortic annulus and/or the aorta (10) or the pulmonary annulusand/or the pulmonary artery.

7. The valve repair system (2) of any of the claims 1-6, wherein the valve component (3) and/or the anchor support (5) are made of nitinol.

8. The valve repair system (2) of any of claims 1-7, wherein the valve (4, 15) is made of biological material.

9. Delivery device (16) comprising:
a valve repair system (2) comprising a valve component (3), in particular a mitral valve component (3) or a tricuspid valve component, holding or adapted to hold a valve (4), in particular a mitral valve (4) or a tricuspid valve and an anchor support (5), in particular an aortic valve or aortic anchor support (5) or a pulmonic valve or pulmonary artery anchor support and a component (6) for linking the valve component (3) and the anchor support (5),
an outer sheath and a guide wire tubing and at least one stent holder constructed to house at least parts of the valve repair system (2),
designed for a minimal invasive introduction into a body and to release the replacement valve system (2) at a designated body part.

10. A delivery device (16) according to claim 9, wherein the device is one delivery catheter constructed to introduce the whole system (2) during the same medical procedure, when in a first step the valve anchor support (5) is able to be deployed in the aorta (10) or the pulmonary artery and in a second step the valve component (3) is able to be deployed in the left or right atrium (7).

11. A delivery device (16) according to claim 9, wherein the device (16) includes several delivery catheters constructed to introduce the system (2) sequentially when, with a first catheter, the valve anchor support (5) is guided and able to be deployed in the aorta (10) or the pulmonary artery and with a second catheter the valve component (3) is guided and able to be deployed between the atrium and the ventricule (7).

12. Method for minimal invasive introduction of a valve repair system (2) comprising a valve component (3), in particular a mitral valve component (3) or a tricuspid valve component, holding or adapted to hold a valve (4), in particular a mitral valve (4) or a tricuspid valve, and an anchor support (5), in particular an aortic valve or aortic anchor support (5) or a pulmonic valve or pulmonary artery anchor support and a component (6) that links or is linkable to the valve component (3) and the anchor support (5) with a delivery device (16) involving the steps:
a. Introducing a delivery device (16) at an inserting point and guiding the delivery device (16) to a designated body part
b. releasing and fixing the anchor support (5) at an anchoring site
c. releasing and deploying the valve component (3) and valve (4)
d. linking the anchor support (3) and the valve component (3),
Wherein step d) is performed before step a), between step a) and b), between step b) and c), or after step c), and wherein step b) is performed before or after step c).
